# EUROPEAN PATENT APPLICATION

(11) **EP 1 197 212 A1**
(43) Date of publication of application: **17.04.2002**
(21) Application number: 00942427.6
(22) Date of filing: 30.06.2000
(51) Int. Cl.: A61K 31/57, A61K 9/70, A61K 47/32

(54) **ADHESIVE PREPARATION FOR PERCUTANEOUS ABSORPTION**

(30) Priority: 01.07.1999 JP 18741599
(71) Applicant: HISAMITSU PHARMACEUTICAL CO. INC., Tosu-shi, Saga-ken 841-0017 (JP)
(72) Inventor: MAKI, Masayoshi, Hisamitsu Pharm. Co. Inc., Tosu-shi, Saga 841-0017 (JP); IKEURA, Yasuhiro, Hisamitsu Pharm. Co. Inc., Tosu-shi, Saga 841-0017 (JP)
(74) Representative: Cresswell, Thomas Anthony
(86) International application number: JP0004361
(87) International publication number: WO0101990

(57) **Abstract**

An adhesive preparation for percutaneous absorption in which high drug permeability at a low drug concentration and satisfactory drug stability have been obtained by regulating the solubility of a drug in a base. The adhesive preparation comprises a base comprising a styrene-isoprene-styrene block copolymer and norethisterone contained in the base. Preferably, the drug is dissolved in the base in an amount of up to 2 wt.% based on the whole base.

## Description

### Technical Field

The present invention relates to an adhesive preparation for percutaneous absorption containing norethisterone dissolved in a base of the adhesive preparation which contains a styrene-isoprene-styrene block copolymer. Further, the present invention relates to an adhesive preparation for percutaneous absorption containing estradiol in a base in an amount of not more than 2 % by weight based on the whole base.

### Background Art

In order to minimize an unnecessary metabolism of estradiol as well as to provide a therapeutic effect as a result of delivering a drug into blood effectively, trials for percutaneous administration have been performed. On the other hand, suppression of an adverse reaction of administered estradiol has also been tried by absorbing progestin percutaneously.

In JP-A-4-342532, a preparation for percutaneous absorption is proposed, which comprises, as main components, active ingredients comprising estradiol and progestin as well as an acrylic adhesive consisting of 2-ethylhexyl acrylate and N-vinyl-2-pyrrolidone as an adhesive. However, this preparation for percutaneous absorption has disadvantages because the acrylic adhesive shows a low drug releasability as well as too strong irritation to the skin to be used for a long term continuous administration. In the indication of existence of patent JP-A-6-510279, a method for adjusting a saturated concentration of drug in a base by admixing a polymer having a different solubility parameter is proposed. However, this proposal has also a problem for a long term administration relating to the skin irritation due to use of an acrylate polymer as a polymer.

In addition, since norethisterone acetate generally admixed for percutaneous administration as a progestin has a higher compatibility with a base compared with norethisterone, a large amount of drug has to be admixed in the styrene-isoprene-styrene block copolymer base or the acrylic base in order to obtain a good permeability. Further, in order to obtain a therapeutically effective amount of permeation, a high concentration of norethisterone acetate should be admixed. Moreover, a large amount of drug is remained in a base of the adhesive preparation after applying to the skin. Consequently, it is less efficient in view of drug permeability and cost-effectiveness.

### Disclosure of the Invention

In consideration of the above problems, an aspect of the present invention is to provide an adhesive preparation for percutaneous absorption intending:
1) a good percutaneous permeability of drug,
2) an effective use of drug in a base.

Further aspect of the present invention is to provide an adhesive preparation for percutaneous absorption, in which a therapeutically effective amount of drug can be correctly and securely released as well as less irritating to the skin after application of the adhesive preparation for percutaneous absorption to the skin of a patient.

As a result of an extensive study in order to solve the above problem, the present inventors found that a good skin permeability of drug and an efficient use of drug in a base could be achieved by admixing norethisterone except for the esters, the derivatives and the salts thereof to a progestin, focusing on a relationship between solubility of a drug in a base and a skin permeability of a drug. Further, the inventors found that estradiol as a estrogen could also be admixed to the above admixure. In addition, the inventors found that an adhesive preparation having a good drug permeability into the skin, a good stability of formulation and a less skin irritation could be obtained by using a styrene-isoprene-styrene block copolymer, a softener and an adhesive agent as base components of an adhesive preparation for percutaneous absorption, and completed the present invention.

The present invention relates to an adhesive preparation for percutaneous absorption containing norethisterone dissolved in a base of the adhesive preparation which contains a styrene-isoprene-styrene block copolymer. More particularly, the present invention relates to the above adhesive preparation for percutaneous absorption having not less than 30 % of releasing rate of norethisterone in water after 24 hours, determined by the drug releasing test (according to the cylinder method described in the USP Drug Release 〈724〉 Test), which is conducted under the conditions of 900 ml of water for test solution; temperature of test solution, 32.0 ±0.5°C; distance from the lowest end of cylinder to the basal inner plane of vessel, 25 ± 2 mm; and rotation of cylinder, 50 rpm.

The present invention further relates to an adhesive preparation for percutaneous absorption comprising further containing estradiol in an amount not more than 2 % by weight based on the whole base.

### Brief Description of Drawing

Fig. 1 shows the results of the releasing tests of estradiol (E2) on the adhesive preparation in Example 4 (▼), Comparative Example 1 (●) and Comparative Example 4(■).

Fig. 2 shows the results of the releasing tests of norethisterone acetate (NETA) and norethisterone (NET) on the adhesive preparations in Example 4 (▼), Comparative Example 1 (●) and Comparative Example 4(■).

Fig. 3 shows the results of the skin permeability tests of estradiol (slant lines), and norethisterone acetate (NETA) and norethisterone (NET) (dots) using hairless mice on the adhesive preparations in Example 4, Comparative Example 1, Comparative Example 2 and Comparative Example 5.

### Best Mode for Carrying Out the Invention

The adhesive preparation for percutaneous absorption of the present invention is a preparation admixed with norethisterone except for the esters, the derivatives and the salts thereof, as a progestin of drug by dissolving in a base of the adhesive preparation. Further, an estrogen such as estradiol and other female sex hormones can be admixed, if necessary.

A high skin permeability, which could not be obtained by the conventional combination of a drug and a base, can be obtained at a low concentration of drug by a combination of norethisterone and base components containing a styrene-isoprene-styrene block copolymer (SIS) of the present invention.

Preferable amount of norethisterone to be admixed as a progestin used in the present invention is the amount at which norethisterone can exist in the base components in soluble state but not crystallized. In the present invention, a definition that the drug exist in soluble state in the base means a condition in which no crystallization of the drug is found in the base, and the drug can exist in any condition in the base so long as no crystallization is observed. A releasing rate of norethisterone in the adhesive preparation for percutaneous absorption of the present invention increases in proportion to an increase in the concentration of norethisterone dissolved in the base components. However, since release of norethisterone deceases depending on an occurrence of crystallization, it is preferable to use at the concentration showing no crystallization.

A preferable amount of norethisterone to be dissolved in the present invention is in an amount not more than 2 % by weight, more preferably 0.1 - 2 % by weight.

Release of norethisterone of the adhesive preparation for percutaneous absorption of the present invention is affected by the basecomponent. Consequently, the solubility of norethisterone can be determined by the drug releasability test. Preferable drug releasing test is a method for determining releasing rate of drug to the water. For example, the releasing test includes a test in which a releasing rate to water is measured after 24 hours under the conditions of test solution, 900 ml; temperature of test solution, 32.0 ± 0.5°C; and rotation of cylinder, 50 rpm. An amount of norethisterone to be dissolved in the adhesive preparation for percutaneous absorption of the present invention is the amount showing not less than 30 %, preferably not less than 40 % of releasing rate after 24 hours by this releasing test.

In the adhesive preparation for percutaneous absorption of the present invention, drugs other than norethisterone can be admixed. Other drugs to be admixed include an estrogen such as estradiol and other female sex hormones. Particularly, since estradiol has similar physico-chemical properties to those of norethisterone, it can be admixed at any ratio depending upon the therapeutic objectives, preferably in amount of 0.1 - 2 % by weight based on the whole base. A ratio of estradiol to norethisterone to be admixed can be determined within the range that the values of estradiol and norethisterone acetate converted to norethisterone show permeabilities similar to those of the known adhesive preparation for percutaneous absorption of estradiol and norethisterone acetate.

An adhesive preparation for percutaneous absorption of the present invention uses a base containing a styrene-isoprene-styrene block copolymer (SIS). The base components preferably include a styrene-isoprene-styrene block copolymer, as well as softener and adhesive resin. Further, the base components of the present invention may include antioxidant, solubilizing agent and absorption enhancer if necessary, in addition to the above described base components.

A styrene-isoprene-styrene block copolymer includes styrene-isoprene-styrene block copolymer (Shell Chem. Corp., trade name: Califrex TR-1107; Califrex TR-1111), styrene-isoprene-styrene block copolymer (Nippon Synthetic Rubber Co. Ltd., trade name: JSR 5000, JSR 5100) and styrene-isoprene-styrene block copolymer (Nippon Zeon Co. Ltd., trade name: Quintac 3421). These polymers can be used in single or in combination of two or more types.

A softener includes liquid paraffin, polybutene, castor oil, cottonseed oil, palm oil, coconut oil and processed oil.

An adhesive resin includes alicyclic saturated hydrocarbon resin (e.g. Alcon P-100, trade name), rosin ester (e.g. KE-311; KE-100; super-ester S-100, trade name), hydrogen alicyclic hydrocarbon (e.g. Escolet 5300, trade name), terpene-based hydrogenated resin (e.g. Clearon P-105, trade name) and hydrogenated rosin ester (e.g. Foral 105, trade name).

The base components of the adhesive preparation for percutaneous absorption of the present invention can further contain, if necessary, other additives in order to maintain adhesion, safety and stability of the base. Concretely, an inorganic filler such as zinc oxide, calcium carbonate, titanium dioxide and silica and a solubilizing agent such as crotamiton, polyisobutylene and dibutyl hydroxytoluene can be admixed properly in an appropriate amount.

Amounts of base components of the present invention to the whole base are as follows:

Styrene-isoprene-styrene block copolymer is 10 - 30 % by weight, preferably 13 - 27 % by weight, more preferably 15 - 25 % by weight; softener is 10 - 60 % by weight, preferably 12 - 55 % by weight, more preferably 15 - 50 % by weight; and adhesive resin is 20 - 60 % by weight, preferably 23 - 57 % by weight, more preferably 25 - 50 % by weight. These compounds can be combined at any ratio within these ranges.

If an amount of the styrene-isoprene-styrene block copolymer is less than the above ranges, cohesive force becomes insufficient, and if exceeds the ranges, it causes problems of poor adhesion as well as less flexibility of the preparation. If an amount of the softener is less than the above ranges, it causes problems of poor adhesion as well as less flexibility of the preparation, and if exceeds the ranges, cohesive force becomes insufficient. If an amount of the adhesive resin is less than the above ranges, it causes a trouble of poor adhesion, and if exceeds the ranges, it causes skin irritation such as corneous exfoliation at removal of the preparation from the skin.

In the base components of the adhesive preparation for percutaneous absorption of the present invention, necessary amounts of other components in addition to the above components may be admixed to prepare a required dosage form. The dosage form of the adhesive preparation for percutaneous absorption of the present invention is preferably plasters, particularly more preferably substantially anhydrous plasters.

The base containing drugs of the adhesive preparation for percutaneous absorption of the present invention is preferably used by spreading on a support mean such as film. The film of the support mean in the present invention should have properties superior in barrier property in order to prevent leakage, volatility and adsorption of the drugs. Further, it is preferable to have a proper flexibility when the adhesive preparation for percutaneous absorption is applied on the skin. A material for the support mean is not specially limited, so long as it clears the above conditions. Concretely, it includes aluminum, ethylene-vinyl acetate copolymer or its saponified polymer, cellulose acetate, cellulose, nylon, polyester, polyethylene, poly(vinylidene chloride), polycarbonate, poly(vinyl alcohol) and polypropylene. These materials are processed to a film or, if necessary, to a laminate with paper or cloth or to a laminated film, as well as a film treated with vapor deposition of aluminum or silica to improve barrier property.

In the adhesive preparation for percutaneous absorption of the present invention, a film for removal liner layer may be provided on the opposite side of the supporting mean. The film for removal liner layer should be able to block leakage and volatility of the drugs from the drug layer during storage of the adhesive preparation for percutaneous absorption. In addition, the removal liner layer has to be removable when it is used on a machine. A material for the removal liner layer is aluminum, cellulose, polyester, polyethylene and polypropylene, and if necessary, these films may be laminated. Further, the surface thereof may be treated with silicon or fluorocarbon, or known additives may be admixed with the liner material to adjust removability or barrier property. In order to make handling for removal easy, the removal liner may be provided with a tab for removal.

Subsequently, a process for manufacturing the adhesive preparation for percutaneous absorption of the present invention will be explained. The adhesive preparation for percutaneous absorption of the present invention can be manufactured, for example, by dissolving all base components excluding drugs under heating, then adding drug components and mixing them homogeneously, and as occasion demands, spreading the mixture on the above described supporting mean followed by covering with a liner, then cutting into a desired form to obtain a product, or spreading the mixture on a film applied with a removal treatment, then transferring the mixture to a suitable supporting mean under a pressure to obtain a product. Alternatively, the adhesive preparation for percutaneous absorption can be manufactured by dissolving all components in an organic solvent such as hexane, toluene and ethyl acetate, then spreading the mixture on the above supporting mean, followed by covering with a liner after removing the organic solvent, then cutting into a desired form to obtain a product, or spreading the mixture on a film applied with a removal treatment, then transferring the mixture to a suitable supporting mean under a pressure after removing the solvent to obtain a product.

Hereinbelow, the adhesive preparation for percutaneous absorption of the present invention will be explained in detail by illustrating examples and experimental examples but the present invention is not limited within these examples.

### Examples

Numerical values in the examples and comparative examples are given by a percent by weight.

| Example 1 | |
|---|---|
| Styrene-isoprene-styrene block copolymer | 10 |
| Liquid paraffin | 60 |
| Adhesive agent (alicyclic satureted hydrocarbon | 20 |
| resin, trade name: Alcon P-100) | |
| Polyisobutylene | 8.8 |
| Dibutyl hydroxytoluene | 1 |
| Estradiol | 0.1 |
| Norethisterone | 0.1 |

A preparation was manufactured in this formulation according to the above manufacturing method and a product was cut in a desired size to obtain a mixed preparation of estradiol and norethisterone.

| Example 2 | |
|---|---|
| Styrene-isoprene-styrene block copolymer | 30 |
| Liquid paraffin | 10 |
| Adhesive agent (rosin ester, trade name: KE-311) | 35 |
| Polyisobutylene | 10 |
| Crotaminton | 10 |
| Dibutyl hydroxytoluene | 1 |
| Estradiol | 2 |
| Norethisterone | 2 |

A preparation was manufactured in this formulation according to the above manufacturing method and a product was cut in a desired size to obtain a mixed preparation of estradiol and norethisterone.

| Example 3 | |
|---|---|
| Styrene-isoprene-styrene block copolymer | 20 |
| Liquid paraffin | 25 |
| Adhesive agent (hydrogenated rosin ester, trade | 30 |
| name: Foral 105) | |
| Polyisobutylene | 12 |
| Crotamiton | 10 |
| Dibutyl hydroxytoluene | 1 |
| Estradiol | 1.5 |
| Norethisterone | 0.5 |

A preparation was manufactured in this formulation according to the above manufacturing method and a product was cut in a desired size to obtain a mixed preparation of estradiol and norethisterone.

| Example 4 | |
|---|---|
| Styrene-isoprene-styrene block copolymer | 25 |
| Liquid paraffin | 30 |
| Adhesive agent (hydrogenated rosin ester, trade | 25 |
| name: Foral 105) | |
| Polyisobutylene | 10 |
| Crotamiton | 8 |
| Dibutyl hydroxytoluene | 1 |
| Estradiol | 0.5 |
| Norethisterone | 0.5 |

A preparation was manufactured in this formulation according to the above manufacturing method and a product was cut in a desired size to obtain a mixed preparation of estradiol and norethisterone.

| Example 5 | |
|---|---|
| Styrene-isoprene-styrene block copolymer | 15 |
| Liquid paraffin | 15 |
| Adhesive agent (hydrogenated rosin ester, trade | 60 |
| name: Foral 105) | |
| Polyisobutylene | 6.6 |
| Hexylene glycol | 1 |
| Dibutyl hydroxytoluene | 1 |
| Estradiol | 0.7 |
| Norethisterone | 0.7 |

A preparation was manufactured in this formulation according to the above manufacturing method and a product was cut in a desired size to obtain a mixed preparation of estradiol and norethisterone.

| Comparative Example 1 | |
|---|---|
| Styrene-isoprene-styrene block copolymer | 25 |
| Liquid paraffin | 30 |
| Adhesive agent (hydrogenated rosin ester, trade | 25 |
| name: Foral 105) | |
| Polyisobutylene | 10 |
| Crotamiton | 8 |
| Dibutyl hydroxytoluene | 1 |
| Estradiol | 0.5 |
| Norethisterone acetate | 0.5 |

A preparation was manufactured in this formulation according to the above manufacturing method and a product was cut in a desired size to obtain a mixed preparation of estradiol and norethisterone acetate.

| Comparative Example 2 | |
|---|---|
| Styrene-isoprene-styrene block copolymer | 25 |
| Liquid paraffin | 24.5 |
| Adhesive agent (hydrogenated rosin ester, trade | 25 |
| name: Foral 105) | |
| Polyisobutylene | 10 |
| Crotamiton | 8 |
| Dibutyl hydroxytoluene | 1 |
| Estradiol | 0.5 |
| Norethisterone acetate | 6 |

A preparation was manufactured in this formulation according to the above manufacturing method and a product was cut in a desired size to obtain a mixed preparation of estradiol and norethisterone acetate.

| Comparative Example 3 | |
|---|---|
| Styrene-isoprene-styrene block copolymer | 25 |
| Liquid paraffin | 25.5 |
| Adhesive agent (hydrogenated rosin ester, trade | 25 |
| name: Foral 105) | |
| Polyisobutylene | 10 |
| Crotamiton | 8 |
| Dibutyl hydroxytoluene | 1 |
| Estradiol | 0.5 |
| Norethisterone acetate | 5 |

A preparation was manufactured in this formulation according to the above manufacturing method and a product was cut in a desired size to obtain a mixed preparation of estradiol and norethisterone acetate.

| Comparative Example 4 | |
|---|---|
| Styrene-isoprene-styrene block copolymer | 30 |
| Liquid paraffin | 10 |
| Adhesive agent (hydrogenated rosin ester, trade | 35 |
| name: Foral 105) | |
| Polyisobutylene | 9 |
| Crotamiton | 10 |
| Dibutyl hydroxytoluene | 1 |
| Estradiol | 2.5 |
| Norethisterone | 2.5 |

A preparation was manufactured in this formulation according to the above manufacturing method and a product was cut in a desired size to obtain a mixed preparation of estradiol and norethisterone.

| Comparative Example 5 (acrylate base) | |
|---|---|
| TS-620 (methyl acrylate - 2-ethylhexyl acrylate copolymer resin emulsion: Nippon Carbide Co., Ltd.) | 91 |
| Crotamiton | 8 |
| Estradiol | 0.5 |
| Norethisterone | 0.5 |

A preparation was manufactured in this formulation according to the above manufacturing method and a product was cut in a desired size to obtain a mixed preparation of estradiol and norethisterone.

### Experimental Example 1. Confirmation on solubility of drugs by crystallization

Appearances of crystals were observed on the test pieces obtained in Examples 1, 2, 3, 4 and 5, and also Comparative Examples 2, 3 and 4 at the initial time, after stored at 25°C for 6 months and after stored at 40°C for 6 months. Results are shown in Table 1.

**Table 1**

| | Initial Time | After the storages for 6 months | |
|---|---|---|---|
| | | 25°C | 40°C |
| Example 1 | No Crystallization | No Crystallization | No Crystallization |
| Example 2 | No Crystallization | No Crystallization | No Crystallization |
| Example 3 | No Crystallization | No Crystallization | No Crystallization |
| Example 4 | No Crystallization | No Crystallization | No Crystallization |
| Example 5 | No Crystallization | No Crystallization | No Crystallization |
| Comparative Example 2 | No Crystallization | Crystallized | Crystallized |
| Comparative Example 3 | No Crystallization | No Crystallization | No Crystallization |
| Comparative Example 4 | No Crystallization | Crystallized | Crystallized |

Although crystallization was not observed at the initial time, but was observed after the storages at 25°C for 6 months and at 40°C for 6 months in Comparative Examples 2 and 4.

According to Comparative Example 2, it was found that admixing of norethisterone acetate should be in an amount less than 6 % by weight for preventing crystallization. Further, since the crystallization was also observed in Comparative Example 4, it was indicated that admixing of norethisterone should be in an amount not more than 2 % by weight for preventing crystallization.

In conclusion, it can be understood that if an amount of norethisterone admixed in the base is in an amount not more than 2 % by weight, norethisterone is completely dissolved in the base.

### Experimental Example 2. ( Releasing test)

Drug releasabilities were tested on the test pieces obtained in Example 4, Comparative Example 1 and Comparative Example 4 (after crystallization) according to the cylinder method described in the USP Drug Release (724) Test under the following conditions.

| | |
|---|---|
| Test solution | 900 ml water |
| Temperature of test solution | 32.0 ± 0.5°C |
| Distance from the lowest end of cylinder to the basal inner plane of vessel | 25 ± 2 mm |
| Rotation of cylinder | 50 rpm |

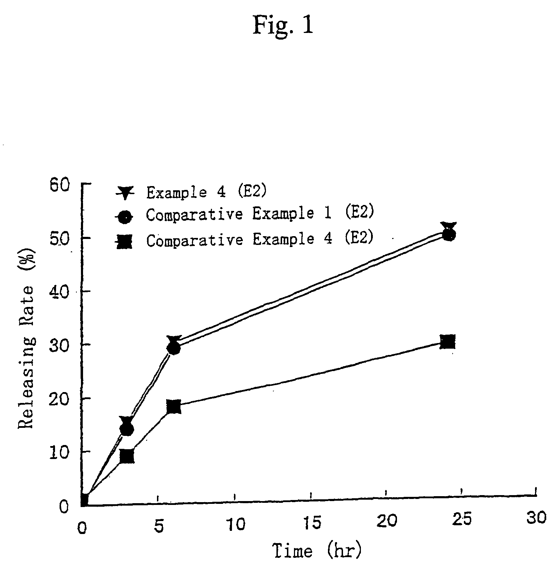

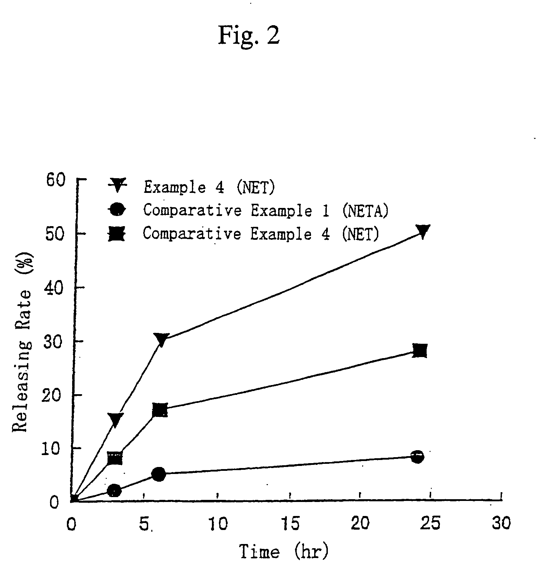

Test results of estradiol (E2) are shown in Fig. 1 and those of norethisterone acetate (NETA) in Comparative Example 1 and norethisterone (NET) in Comparative Example 4 are shown in Fig. 2, respectively. As a progestin, 0.5 % of norethisterone in Example 4 and 0.5 % of norethisterone acetate in Comparative Example 1 were admixed, respectively. In spite of the identical concentrations of the drugs were used in the tests, a good releasing rate of progestin after 24 hours showing 30 % or more could be obtained only with the test piece from Example 4. In Comparative Example 4, decreases in the releases of drugs caused by crystallizations of estradiol and norethisterone were observed.

### Experimental Example 3. (Skin permeability test)

Dorsal skin permeability tests of hairless mice (7 weeks old, female) were conducted at 37°C using Franz's diffusion cell on the test pieces obtained in Example 4, Comparative Example 1, Comparative Example 2 and Comparative Example 5. After initiation of the test, a receptor solution was collected at certain times, and immediately thereafter, the receptor solution was supplemented, then an amount of the drug permeated to the collected receptor solution was assayed by means of high performance liquid chromatography. Numbers of samples in each test piece were 3 pieces, respectively.

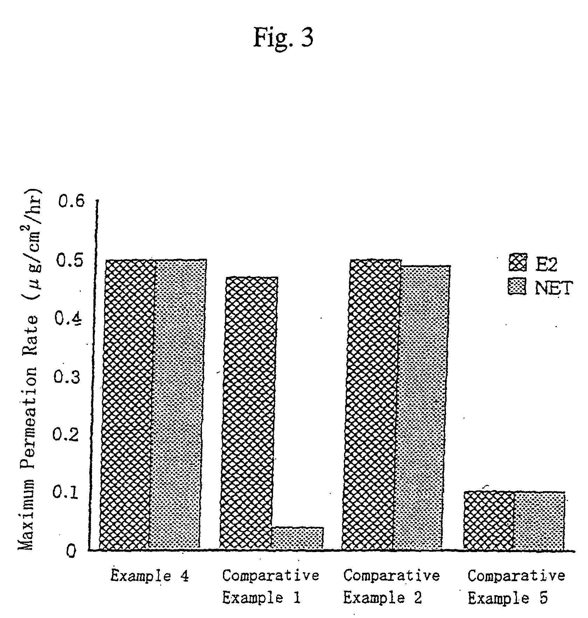

Results are shown in Fig. 3. In Fig. 3, columns with slant lines show estradiol (E2) and those of dots show norethisterone (NET) or norethisterone acetate (NETA), respectively. With respective to Example 4 and Comparative Example 2, despite of the high concentration of progestin in Comparative Example 2, the skin permeabilities were in the equivalent level. In Comparative Example 1 wherein the progestin concentration was made equal to that in Example 4, the permeability of norethisterone acetate resulted in a considerably low level. The drugs in Comparative Example 5 using the acrylate base exhibited much lower drug permeability than the drugs in Example 4.

A distribution coefficient between a skin and a base is known as one of factors to determine a skin permeation rate of a drug. A high distribution coefficient can be obtained by enlarging the difference in solubility parameters between a drug and a base component. Consequently, it is necessary to select a base having a solubility parameter distant from the drug and to apply a drug having a solubility parameter close to the skin. In the adhesive preparation for percutaneous absorption of the present invention, the superior results seemed to be obtained in the skin permeability test, since a high distribution coefficient could be obtained by combining with the base.

### Industrial Applicability

The adhesive preparation for percutaneous absorption of the present invention has an effect showing a high skin permeability at a low drug concentration. In addition, as the result of using norethisterone except for the esters, the derivatives and the salts thereof, excess dissolving of the drug to a base can be prevented as well as maintaining proper permeability. Further, with the adhesive preparation for percutaneous absorption of the present invention, programmed amounts of progestin and estrogen can be applied to a patient accurately and securely at the lower drug contents than in the conventional adhesive preparation for percutaneous absorption.

Moreover, since the adhesive preparation for percutaneous absorption of the present invention has a high degree of freedom in its composition, a proper design of dosage form in compliance with conditions can be made by considering its efficacy and stability.

## Claims

1. An adhesive preparation for percutaneous absorption containing norethisterone dissolved in a base of the adhesive preparation which contains a styrene-isoprene-styrene block copolymer.

2. The adhesive preparation for percutaneous absorption according to claim 1, wherein an amount of norethisterone to be dissolved is the amount to show the releasing rate in water being not less than 30 % after 24 hours determined by the drug releasing test (according to the cylinder method described in the USP Drug Release 〈724〉 Test), which is conducted under the conditions of water for test solution, 900 ml; temperature of test solution, 32.0 ±0.5°C; distance from the lowest end of cylinder to the basal inner plane of vessel 25 ± 2 mm; and rotation of cylinder, 50 rpm.

3. The adhesive preparation for percutaneous absorption according to any of claims 1 - 2, wherein an amount of norethisterone to be dissolved is in the amount not more than 2 % by weight based on the whole base.

4. The adhesive preparation for percutaneous absorption according to any of claims 1 - 3, comprising further containing estradiol in an amount not more than 2 % by weight based on the whole base.

5. The adhesive preparation for percutaneous absorption according to any of claims 1 - 4, wherein the adhesive preparation containing a styrene-isoprene-styrene block copolymer comprises 10 - 30 % by weight of a styrene-isoprene-styrene block copolymer, 10 - 60 % by weight of a softener and 20 - 60 % by weight of an adhesive resin based on the whole base.
